# DEMANDE DE BREVET EUROPEEN

(11) **EP 4 760 724 A1**
(43) Date de publication de la demande: **17.06.2026**
(21) Numéro de dépôt: 25155338.4
(22) Date de dépôt: 31.01.2025
(51) Int. Cl.: G16B 20/00, G16B 30/10

(54) **PROCÉDÉ ET DISPOSITIF DE DÉTERMINATION AU PLUS TÔT D'UN CARACTÈRE PHÉNOTYPIQUE À PARTIR D'UN FLUX DE SÉQUENCES GÉNOMIQUES**

(30) Priorité: 12.12.2024 EP 24219364
(71) Demandeur: bioMérieux, 69280 Marcy l'Etoile (FR)
(72) Inventeur: EL AZAMI, Meriem, 69210 L'Arbresle (FR); MAHE, Pierre, 59800 LILLE (FR)
(74) Mandataire: bioMérieux PI Groupement mandataires

(57) **Abrégé**

Un procédé de prédiction phénotypique d'un organisme en fonction de son génome, comprend un séquençage du génome de manière à produire des reads, délivrés en temps réel et/ou par lots à intervalles réguliers ou périodiques, et un traitement itératif comprenant une mise à jour d'un ensemble de reads en fonction des nouveaux reads délivrés, une détection des k-mers présents dans l'ensemble, une prédiction phénotypique en fonction des k-mers détectés; et la délivrance de la prédiction lorsqu'un critère d'arrêt est atteint.

Le critère d'arrêt est atteint si la variation du nombre de k-mers détectés est stable pendant un premier nombre prédéterminé d'itérations et si la variation du caractère phénotypique est stable pendant un second nombre d'itérations et si la variation de la profondeur de séquençage entre deux itérations est supérieure à un seuil prédéterminé.

## Description

### DOMAINE DE L'INVENTION

L'invention a trait au domaine de la caractérisation microbienne, en particulier la détermination de traits phénotypiques de microorganismes, sur la base du séquençage de leurs génomes réalisé au moyen d'un séquenceur délivrant des séquences en temps réel ou par lot de manière fréquente ou régulière.

### ETAT DE LA TECHNIQUE

La diminution rapide des coûts et l'amélioration significative des performances des séquenceurs de troisième génération, comme les séquenceurs par SBS de la société Illumina Inc. ou les séquenceurs par nanopores de la société Oxford Nanopore Technologies, ont favorisé le développement rapide de la science dite "genomic-to-phenotypic". Cette discipline vise à prédire les caractéristiques phénotypiques des organismes en se basant sur leurs génomes. En microbiologie par exemple, il est désormais possible de prédire si une bactérie résiste ou non à un antibiotique par analyse génomique. Cette approche vise à compléter, voire remplacer, les méthodes phénotypiques traditionnelles à base de culture microbienne qui délivrent leurs résultats trop lentement au regard des pathologies graves comme le sepsis.

Les séquenceurs actuels présentent néanmoins une limitation notable qui les empêche de surpasser les méthodes traditionnelles. En effet, le temps de séquençage requis pour obtenir des résultats fiables avec des erreurs de séquençage compatibles avec la discipline génomique-phénotypique est encore trop long, se situant au mieux entre un à deux jours. Tout d'abord, les séquenceurs de type SBS ou équivalent, délivrent leur résultat en une seule fois, à la toute fin du séquençage. Ils offrent néanmoins l'avantage de présenter un taux d'erreur de séquençage extrêmement bas, ce taux devenant quasi nul une fois que les reads (i.e. les séquences d'acides nucléiques numériques produites par un séquenceur) qu'ils produisent sont assemblés. À la différence des séquenceurs par courts fragments, le séquençage par nanopores délivre ses reads de manière continue, soit en temps réel, soit par lots à intervalles réguliers. Cela permet une analyse immédiate, et l'utilisateur peut, en théorie, interrompre le séquençage dès que le niveau de confiance souhaité est atteint, optimisant ainsi les délais. Cependant, les erreurs de séquençage par nanopores sont plus fréquentes (3%-5% contre 0,1% pour les séquenceurs SBS), de sorte qu'il est aujourd'hui nécessaire d'obtenir des profondeurs de séquençage d'au moins 50x afin d'obtenir le niveau de confiance requis. Finalement, le gain de temps pour cette analyse au fil de l'eau est minime.

### EXPOSE DE L'INVENTION

Le but de la présente invention est de fournir un procédé et un système de caractérisations phénotypique d'un organisme sur base de son matériel nucléique (ADN, ARN, protéique...), matériel numérisé par un séquenceur délivrant les reads au fil de l'eau, soit en temps réel soit par lots à intervalles réguliers et périodiques, permettant de réduire :
- la durée nécessaire pour obtenir un résultat aussi fiable que celui produit par séquençage de grande profondeur et/ou de longue durée ;
- la quantité de reads nécessaire pour obtenir un tel résultat, ce qui permet (a) d'économiser des ressources informatiques (quantité de mémoire, nombre de processeurs, etc.) et/ou du temps de calcul grâce à un volume réduit de données à traiter, (b) d'économiser des réactifs utilisés pour le séquençage (comme par exemple les réactifs pour la préparation de librairies) et/ou de limiter l'usure de la plateforme de séquençage et de ses consommables (par exemple, des cellules de séquençage ou « flow cell » en anglais) en séquençant uniquement ce qui est nécessaire pour atteindre le résultat.

A cet effet, l'invention a pour objet un procédé de prédiction d'un caractère phénotypique d'un organisme en fonction de son génome, comprenant :
- un séquençage dudit génome de manière à produire des séquences d'acides nucléiques numériques représentatives dudit génome, ci-après "reads", ledit séquençage étant configuré pour délivrer les reads a) en temps réel et/ou b) par lots à intervalles réguliers ou périodiques, lesdits reads étant mémorisés dans une mémoire informatique ;
- un traitement itératif mis en oeuvre par ordinateur, comprenant pour chaque itération:
   - une mise à jour d'un ensemble de reads en fonction des reads délivrés en temps réel ou par lots ;
   - un calcul d'une profondeur de séquençage dudit génome en fonction dudit ensemble de reads ;
   - une détection de séquences d'acides nucléiques numériques de référence de longueur prédéterminée, ci-après "k-mers", dans ledit ensemble de reads, un k-mer étant détecté lorsque son nombre d'occurrences dans ledit ensemble est supérieure à une fraction prédéterminée de la profondeur de séquençage calculée;
   - une prédiction du caractère phénotypique en fonction des k-mers détectés dans ledit ensemble de reads; et
   - un calcul d'un critère d'arrêt et une mémorisation du caractère phénotypique prédit en tant que caractère phénotypique final lorsque ledit critère d'arrêt est atteint,

Selon l'invention, le calcul du critère d'arrêt comprend le calcul de la variation de la profondeur de séquençage calculée entre deux itérations, de la variation du nombre de k-mers détectés entre deux itérations et de la variation du caractère phénotypique prédit entre deux itérations, et le critère d'arrêt est atteint :
A. si la variation du nombre de k-mers détectés est stable pendant un premier nombre prédéterminé d'itérations ; et
B. à la suite de la stabilité de la variation du nombre de k-mers détectés pendant le premier nombre d'itérations :
   B.1. si la variation du caractère phénotypique est stable pendant un second nombre d'itérations; et
   B.2. si la variation de la profondeur de séquençage calculées pendant le second nombre prédéterminé d'itérations est supérieure à un seuil prédéterminé.

En d'autres termes, pour accélérer la délivrance de la prédiction phénotypique, l'invention permet a) de détecter au plus tôt lorsque cette dernière est susceptible de se stabiliser sur un résultat conforme au contenu du génome, b) d'identifier cette stabilité de manière rapide et robuste.

Au début du séquençage, tant le nombre de kmers détectés que les résultats de la prédiction fluctuent considérablement en raison de la faible profondeur de séquençage. Par conséquent, si un critère d'arrêt se base uniquement sur la prédiction phénotypique, il est nécessaire de prévoir une large fenêtre d'itérations pour l'analyse de stabilité afin éviter qu'une stabilisation passagère soit interprétée comme un résultat attendu. Cependant, plus la fenêtre est large, plus grand est le délai de délivrance de résultats déjà stabilisés. L'invention propose alors une première stabilisation du nombre de kmers détectés afin d'identifier la fin effective ou imminente de cette période instable, ce qui permet de réaliser l'analyse de stabilité de la prédiction sur une fenêtre plus réduite. De plus, en raison du bruit de séquençage potentiellement élevé (jusqu'à 5% sur certaines plateformes), le nombre de kmers peut varier pour des profondeurs de séquençage plus importantes et ce même après la phase initiale. Pour que ce bruit n'affecte pas la stabilité de la caractérisation phénotypique, la fenêtre de stabilité est définie de manière à lisser ce bruit sans altérer le résultat final. A cette fin, une profondeur de séquençage minimale est imposée entre deux itérations de la fenêtre ou sur la fenêtre totale. Ainsi, le résultat final est robuste et peut être communiqué avec confiance.

A titre d'exemple, en choisissant le premier nombre d'itération égale à 1, le second nombre d'itération à 4, et l'incrément de profondeur de séquençage égal à 3x, les résultats de la prédiction sont délivrés aux alentours de 20x. Ceci constitue un gain de 30x par rapport un arrêt du séquençage réalisé classiquement autour des 50x.

L'arrêt complet du séquençage peut néanmoins être différé en fonction des résultats d'autres analyses réalisées en parallèle, par multiplexage notamment. Dans ce type d'analyse, l'arrêt du séquençage est réalisé lorsque tous les échantillons multiplexés ont atteint leur stabilité en termes de prédiction.

Selon un mode de réalisation, la fraction prédéterminée de la profondeur de séquençage est comprise entre 0,08 et 0,18. Selon un mode de réalisation, le premier nombre d'itérations est égal à 1. Selon un mode de réalisation, le second nombre d'itérations est supérieur ou égal à 4. Selon un mode de réalisation, le seuil est supérieur ou égal à 3x.

Selon un mode de réalisation, la variation du nombre de k-mers détectés est stable lorsqu'elle est inférieure en valeur absolue à un premier seuil de stabilité, et la variation du caractère phénotypique est stable lorsqu'elle est inférieure à un second seuil de stabilité prédéterminé, et le premier et second seuil sont inférieurs ou égaux à 0.1.

Selon un mode de réalisation, le séquençage est configuré pour délivrer des reads d'une longueur moyenne supérieure à 500kb.

Selon un mode de réalisation, les reads ne sont pas assemblés.

Selon un mode de réalisation, préalablement au séquençage, le procédé comporte la production d'un échantillon à partir d'un isolat de microorganisme, le séquençage étant mis en oeuvre sur ledit échantillon.

Selon un mode de réalisation, l'organisme est microorganismes, et le caractère phénotypique est un profil de résistance ou de sensibilité du microorganisme à au moins un anti-microbien. En particulier, le microorganisme est prélevé sur un patient ou un animal suspecté d'une infection microbienne, et le procédé comprend la détermination d'une thérapie antimicrobienne en fonction dudit profil et l'administration de ladite thérapie au patient ou à l'animal.

Selon un mode de réalisation, lorsque le critère d'arrêt est atteint le séquençage est stoppé et/ou le caractère phénotypique final est affiché sur un écran.

L'invention a également pour objet un système de prédiction d'un caractère phénotypique d'un organisme en fonction de son génome, comprenant :
- un séquenceur d'acides nucléiques, ledit séqueneur étant configurée pour séquencer ledit génome de manière à produire des séquences d'acides nucléiques numériques représentatives dudit génome, ci-après "reads", ledit séquenceur étant configuré pour délivrer les reads a) en temps réel et/ou b) par lots à intervalles réguliers ou périodiques, lesdits reads étant mémorisés dans une mémoire informatique ;
- une unité de traitement d'information connectée au séquenceur pour recevoir lesdits reads et comprenant un ou plusieurs microprocesseurs et une mémoire informatique mémorisant des instructions informatiques qui, lorsqu'elles sont exécutées par le ou les microprocesseurs, mettent en oeuvre un traitement itératif, comprenant pour chaque itération:
   - une mise à jour d'un ensemble de reads en fonction des reads délivrés en temps réel ou par lots ;
   - un calcul d'une profondeur de séquençage dudit génome en fonction dudit ensemble de reads ;
   - une détection de séquences d'acides nucléiques numériques de référence de longueur prédéterminée, ci-après "k-mers", dans ledit ensemble de reads, un k-mer étant détecté lorsque son nombre d'occurrences dans ledit ensemble est supérieure à une fraction prédéterminée de la profondeur de séquençage calculée;
   - une prédiction du caractère phénotypique en fonction des k-mers détectés dans ledit ensemble de reads; et
   - un calcul d'un critère d'arrêt et une mémorisation du caractère phénotypique prédit en tant que caractère phénotypique final lorsque ledit critère d'arrêt est atteint.

Selon l'invention, le calcul du critère d'arrêt comprend le calcul de la variation de la profondeur de séquençage calculée entre deux itérations, de la variation du nombre de k-mers détectés entre deux itérations et de la variation du caractère phénotypique prédit entre deux itérations, et le critère d'arrêt est atteint :
A. si la variation du nombre de k-mers détectés est stable pendant un premier nombre prédéterminé d'itérations ; et
B. à la suite de la stabilité de la variation du nombre de k-mers détectés pendant le premier nombre d'itérations :
   B.1. si la variation du caractère phénotypique est stable pendant un second nombre d'itérations; et
   B.2. si la variation de la profondeur de séquençage calculées pendant le second nombre prédéterminé d'itérations est supérieure à un seuil prédéterminé.

Selon un mode de réalisation, la mémoire informatique mémorisant des instructions informatiques qui, lorsqu'elles sont exécutées par le ou les microprocesseurs, mettent en oeuvre un procédé du type précité.

L'invention a également pour objet un produit programme d'ordinateur comprenant une mémoire informatique mémorisant des instructions informatiques qui, lorsqu'elles sont exécutées par un ou plusieurs microprocesseurs, mettent en oeuvre le traitement itératif décrit ci-avant.

L'invention a également pour objet un procédé de production d'une mémoire informatique pour la prédiction d'un caractère phénotypique d'un organisme en fonction de son génome comprenant :
- la mise à jour itérative d'un ensemble de séquences d'acides nucléiques numériques représentatives dudit génome, ci-après "reads", en fonction de nouveaux reads représentatifs dudit génome;
- l'exploration mise en oeuvre par informatique d'un espace défini par au moins un premier nombre d'itérations, un second nombre d'itérations et un seuil, ci-après « triplet », l'exploration comprenant pour chaque valeur d'un ensemble de triplets dudit espace (a) un calcul d'une profondeur de séquençage dudit génome en fonction dudit ensemble de reads, une détection de séquences d'acides nucléiques numériques de référence de longueur prédéterminée, ci-après "k-mers", dans ledit ensemble de reads, un k-mer étant détecté lorsque son nombre d'occurrences dans ledit ensemble est supérieure à une fraction prédéterminée de la profondeur de séquençage calculée, (b) une prédiction du caractère phénotypique en fonction des k-mers détectés dans ledit ensemble de reads, et (c) un calcul d'un critère d'arrêt, le calcul du critère d'arrêt comprenant le calcul de la variation de la profondeur de séquençage calculée entre deux itérations, de la variation du nombre de k-mers détectés entre deux itérations et de la variation du caractère phénotypique prédit entre deux itérations ;
- la mémorisation dans la mémoire informatique (a) d'un triplet en tant que premier nombre d'itérations, second nombre d'itérations, et seuil et (b) d'instructions informatiques qui, lorsqu'elles sont exécutées par un ou plusieurs microprocesseurs, mettent en oeuvre le traitement itératif selon l'une quelconque des revendications 1 à 12, lorsque ledit triplet rempli les conditions I et II suivantes:
   I.
      A. si la variation du nombre de k-mers détectés est stable pendant le premier nombre prédéterminé d'itérations ; et
      B. à la suite de la stabilité de la variation du nombre de k-mers détectés pendant le premier nombre d'itérations :
         B.1. si la variation du caractère phénotypique est stable pendant le second nombre d'itérations; et
         B.2. si la variation de la profondeur de séquençage calculées pendant le second nombre prédéterminé d'itérations est supérieure au seuil,
   II. lorsque la condition I est remplie :
      C. 1. si les k-mers détectés sont également détectés dans un génome de référence de l'organisme ;
      C.2. et si le caractère phénotypique détecté est identique à un caractère phénotypique de référence pour l'organisme.

### BREVE DESCRIPTION DES FIGURES

L'invention sera mieux comprise à la lecture de la description qui va suivre, donnée uniquement à titre d'exemple, et faire en relation avec les dessins annexés, dans lesquels des références identiques désignent des éléments identiques ou analogues, et dans lesquels
- la **figure 1** illustre une architecture informatique et matériel pour la mise en oeuvre d'une analyse microbiologique selon l'invention ;
- la **figure 2** illustre l'invention dans le cadre d'un flux de travail (« worflow ») complet d'analyse microbiologique ;
- la **figure 3** illustre une prédiction bi-classes selon l'invention, correspondant à la résistance ou la sensibilité d'une souche d'espèce bactérienne donnée à un antibiotique donné, avec un zoom sur la période initiale d'instabilité et un zoom sur la fenêtre d'analyse de stabilité de la prédiction ;
- la **figure 4** illustre un procédé de détermination de la valeur de paramètres de stabilité selon l'invention ;
- la **figure 5** illustre la courbe ROC pour une souche donnée et pour différentes valeurs de X^{kmer} .
- les **figures 6A** et **6B** illustrent des valeurs d'AUC pour les valeurs optimales de la fraction X^{kmer} pour un ensemble de 55 souches bactériennes en fonction de la profondeur de séquençage moyenne, 3 itérations étant représentées ;
- la **figure 7** illustre une distribution de la fraction X^{kmer} pour les 55 souches bactériennes en fonction d'intervalles de profondeurs moyennes de séquençage ;
- la **figure 8** illustre sous forme de box plot la profondeur de séquençage moyenne à laquelle est délivré la prédiction phénotypique selon l'invention ;
- la **figure 9** illustre le gain de temps entre une prédiction métagénomique à 50x et une prédiction métagénomiques selon l'invention ;
- la **figure 10** illustre ce gain de temps pour les 215 souches testées pour leur résistance/sensibilité à un antibiotique ;
- la **figure 11** est une matrice de confusion entre une prédiction métagénomique selon l'invention et une prédiction métagénomique à 50x ;
- la **figure 12** illustre la profondeur de séquençage moyenne à laquelle la prédiction métagénomique selon l'invention est détectée stabilisé en donc délivrée ; et
- la **figure 13** illustre une variante d'architecture logicielle et matérielle pour la mise en oeuvre selon l'invention.

### DESCRIPTION DETAILLEE DE L'INVENTION

### MODE DE REALISATION

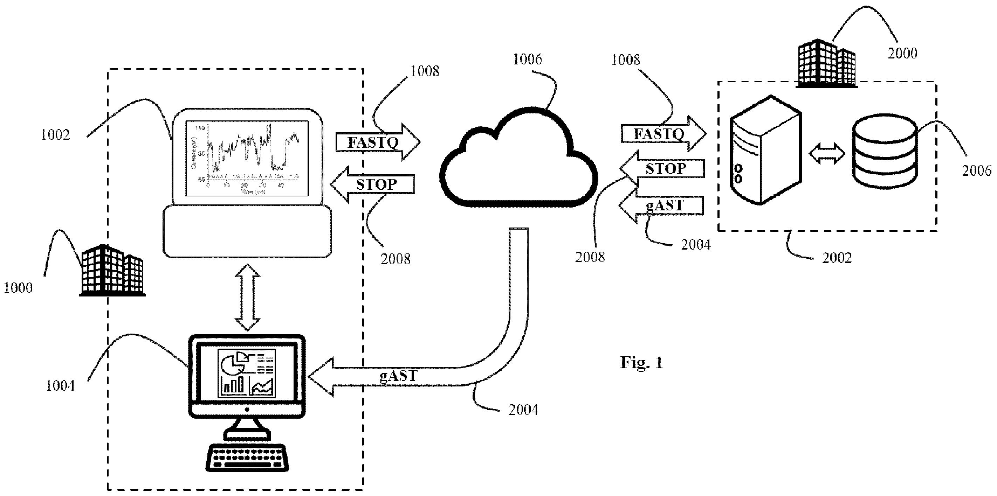

La **figure 1** illustre une architecture informatique et matériel pour la mise en oeuvre d'une analyse microbiologique selon l'invention.

Une première organisation **1000,** par exemple un laboratoire d'analyse microbiologique d'un centre hospitalier, héberge un séquenceur **1002.** Ce dernier comporte une plateforme de séquençage, générant des signaux bruts en fonction de bases nucléiques contenues dans un échantillon en cours d'analyse ainsi qu'une première unité de traitement informatique, connectée à la plateforme (par exemple réseau si cette unité est déportée par exemple ou par fil lorsqu'elle est dans le même châssis que la plateforme comme illustré dans cette figure) et traduisant ces signaux bruts en séquences numériques d'acides nucléiques ou « reads » (cette transformation des signaux étant usuellement appelée « base calling »). A titre d'exemple, la plateforme de séquençage est un Gridion et la flow cell et la préparation de la librairie sont celles commercialisé sous la dénomination « R9.4 », commercialisés par la société Oxford Nanopore Technologies.

Le séquenceur **1002** est par ailleurs connecté à une deuxième unité de traitement **1004** qui met en oeuvre la partie front-end d'une analyse de type SaaS (« Software-as-a-Service) hébergée en Cloud. Une seconde organisation **2000,** par exemple la Demanderesse, héberge un serveur informatique **2002.** Ce serveur est connecté à distance au travers du réseau Internet **1006** au séquenceur **1002** pour en recevoir les reads **1008** (sous forme de fichier fastQ par exemple), et mettre en oeuvre leur traitement afin de prédire une ou plusieurs caractéristiques phénotypiques **2004,** par exemple un antibiogramme « gAST ». Le serveur **2002,** également connecté à l'unité de traitement **1004,** lui communique cette prédiction pour son affichage et son stockage en local. Le serveur **2002** comporte par ailleurs une base de données de référence **2006** pour la mise en oeuvre de cette prédiction, telle que décrite ci-après. Enfin, le serveur **2002** surveille la stabilité de la prédiction afin de détecter au plus tôt cette dernière et envoyer, soit au séquenceur **1002** soit à l'unité **1004** qui alors contrôle le séquenceur, un signal d'arrêt **2008** (« stop ») du séquençage et le résultat définitif de la prédiction. L'unité informatique embarquée dans le séquenceur et la seconde unité **1004** ont par exemple des architectures informatiques classiques de type ordinateur personnel. Le serveur **2002** dispose préférentiellement des capacités de calculs plus importantes en raison de la quantité de données à traiter, par exemple sous la forme de noeuds de calcul.

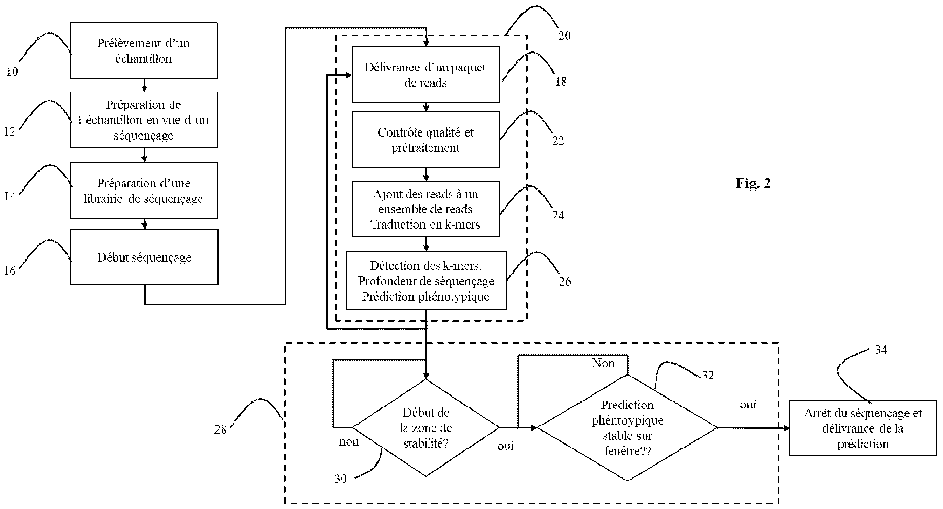

La **figure 2** illustre l'invention dans le cadre d'un flux de travail (« worflow ») complet d'analyse microbiologique, depuis le prélèvement d'un échantillon, sur un patient ou un animal suspecté d'une infection bactérienne par exemple, jusqu'à la délivrance de l'antibiogramme des souches bactériennes dans l'échantillon prélevé. S'il est décrit un mode de réalisation de l'invention appliqué à la prédiction de la résistance ou de la sensibilité d'une souche bactérienne à un antibiotique, on comprend que cette application ne restreint pas l'invention, qui peut être utilisée pour tout type de caractérisation phénotypique, telles que la prédiction de son espèce, la prédiction d'un virulome, d'un sérotype, la similarité génomique avec une autre souche microbienne (ou "typage"), etc.

Cette dernière débute, en **10,** par le prélèvement de l'échantillon biologique qui subit, en **12,** une première phase de préparation en vue d'isoler une souche bactérienne avec une biomasse suffisante pour le séquençage, d'extraire le matériel génétique des cellules correspondant à cette souche (par lyse chimique ou mécanique par exemple), et de concentrer et purifier ce matériel. Une deuxième préparation de l'échantillon ainsi produit consiste ensuite, en **14,** à préparer le matériel génétique en vue de son séquençage par le séquenceur **1002,** étape connue sous le nom de « library préparation ».

Une fois l'échantillon final chargé dans un séquenceur **200** et une fois le séquençage débuté en **16,** le séquenceur **1002** délivre, en **18,** des paquets de reads **1008** à pas de temps fixe ou à intervalle de temps réguliers (par exemple, les paquets sont de tailles fixes, un paquet étant envoyé lorsqu'il est plein) pour leur stockage et analyse par le serveur **2002.** La réception d'un paquet de reads par le serveur **2002** débute une nouvelle itération de traitement informatique **20,** traitement comportant une étape optionnelle **22** de prétraitement des reads en vue d'en caractériser leur qualité et de filtrer des reads jugés de mauvaise qualité (en tant qu'exemples non limitatifs : un filtrage sur des reads ayant une longueur inférieure à seuil prédéfini, un filtrage des reads ayant un score de qualité inférieur à un seuil prédéfini, un filtrage des reads qui correspondent aux adapteurs utilisés lors de la préparation de la librairie...).

Les reads prétraités sont alors stockés, en **24,** dans un ensemble de reads constitués de reads filtrés issus des itérations précédentes. Ensuite, les nouveaux reads ajoutés sont traduits en k-mers, c'est-à-dire en séquences nucléiques de longueur fixe, nommée « *k* », comprise de préférence entre 15 et 50, par exemple 31. Cette longueur permet d'obtenir des k-mers distincts des uns des autres tout en permettant de décrire avec robustesse la diversité génétique microbienne. Cette traduction est par exemple réalisée un faisant glisser par pas de 1 une fenêtre de longueurs *k* sur chaque read est en mémorisant chaque k-mers ainsi détecté dans un ensemble de k-mers comportant des k-mers des itérations précédentes.

Le procédé se poursuit, en **26,** par la détection des k-mers effectivement compris dans le matériel génétique de l'échantillon en cours de test. Pour ce faire, une profondeur de séquençage est calculée par le serveur **2002,** soit directement à partir de l'ensemble de reads, soit directement sur l'ensemble de k-mers. A titre d'exemple, un pan-génome assemblé de l'espèce de la souche bactérienne en cours de caractérisation est mémorisé dans la base de données de référence **2006** et les k-mers sont alignés sur ce génome de référence (par exemple avec une correspondance parfaite à l'aide d'outils tel que *Mummer4* disponible à l'adresse https://mummer4.github.io/, et décrit dans l'article *"*MUMmer4: A fast and versatile genome alignment system" G. Marçais et al., PLoS computational biology (2018)). Une fois l'alignement réalisé, une profondeur de séquençage moyenne, ou locale telle que décrite dans la demande de brevet de numéro de dépôt EP22166649, est calculée. Les k-mers de l'ensemble de k-mers sont ensuite énumérés, et si le nombre d'occurrences d'un k-mer dépasse un seuil égal à une fraction X^{kmer} de la profondeur de séquençage calculée, de manière avantageuse une fraction X^{kmer} comprise entre 0,08 et 0,18, alors le serveur **2002** détecte ce k-mer comme effectivement présent dans le matériel génétique séquencé. Une fois les k-mers détectés, une prédiction phénotypique est mise en oeuvre par le serveur **2002,** par exemple la prédiction d'un antibiogramme telle que décrite dans les demandes WO2021180771 et WO2021180768, prédiction qui est retourné à l'unité **1004.** On notera que cette prédiction est réalisée directement sur les k-mers, sans que les reads ne soient jamais assemblés en contigs, l'assemblage étant une opération très coûteuse en temps et en ressources informatiques.

En parallèle du traitement itératif **20,** le serveur **2002** met en oeuvre une tâche informatique d'analyse de la stabilité de la prédiction phénotypique. A cet effet, ce traitement met en oeuvre un premier test de stabilité **30** sur le nombre de k-mers détectés, dans le but de détecter la fin effective ou imminente de la première phase d'instabilité. Notamment, si la variation du nombre de k-mers détectés entre deux itérations successives, exprimé en pourcentage, est inférieure en valeur absolue à un seuil S^{kmer} pendant N^{kmer} itérations successives, alors ladite fin est identifiée. De manière avantageuse, le seuil S^{kmer} est égal à 0,1 et le nombre d'itérations N^{kmer} est égal à 1.

Une fois ce premier critère de stabilité rempli, le serveur **2002** met en oeuvre une deuxième analyse de stabilité **32** sur la prédiction phénotypique. Si cette dernière est stable sur une fenêtre de N^{pred} itérations successives, et que l'accroissement de la profondeur de séquençage moyenne entre deux itérations successives de cette fenêtre est supérieur à un seuil ΔX alors le serveur **2002** détermine que la prédiction est stable.

Il existe de multiples façons de calculer la variation de la prédiction. Cette dernière délivre un vecteur de sortie (un composant par couple de bactérie-antibiotique testé par exemple), qu'il soit discret (comme par exemple la résistance ou la sensibilité à un antibiotique) ou continu (comme par exemple la concentration minimale inhibitrice d'une souche bactérienne à un antibiotique).

La variation est par exemple calculée comme une norme euclidienne normalisée de la différence entre deux vecteurs successifs délivrés par la prédiction. La variation est alors jugée stable si la norme calculée est inférieure à un seuil S^{Pred}. Ou bien pour une prédiction multi-étiquettes, sa variation est égale au nombre de sorties de la prédiction qui ont changés depuis l'itération précédente, et si la variation de la prédiction entre deux itérations successives, exprimé en pourcentage, est inférieure en valeur absolue à un seuil S^{pred} pendant N^{pred} itérations successives, alors la prédiction est jugée stable. De manière avantageuse, le nombre d'itération N^{pred} est égal à 4, le seuil S^{pred} est égal 0.1, et l'accroissement de profondeur de séquençage moyenne de séquençage sur la fenêtre est avantageusement fixé à 3x, ce qui permet de lisser efficacement le bruit de séquençage des technologies à base de nanopores biologiques que ceux développés par la société Oxford Nanopore Technology, et donc rendre à résultat robuste.

Le serveur **2002** envoie alors, en **34,** un signal d'arrêt du séquençage au séquenceur **2002** qui stoppe le séquençage en cours, et envoie la prédiction finale à l'unité de traitement **1004.** Un clinicien en est averti, par email, SMS ou autres, et détermine une antibiothérapie pour le patient en fonction de cette prédiction, antibiothérapie délivrée au patient.

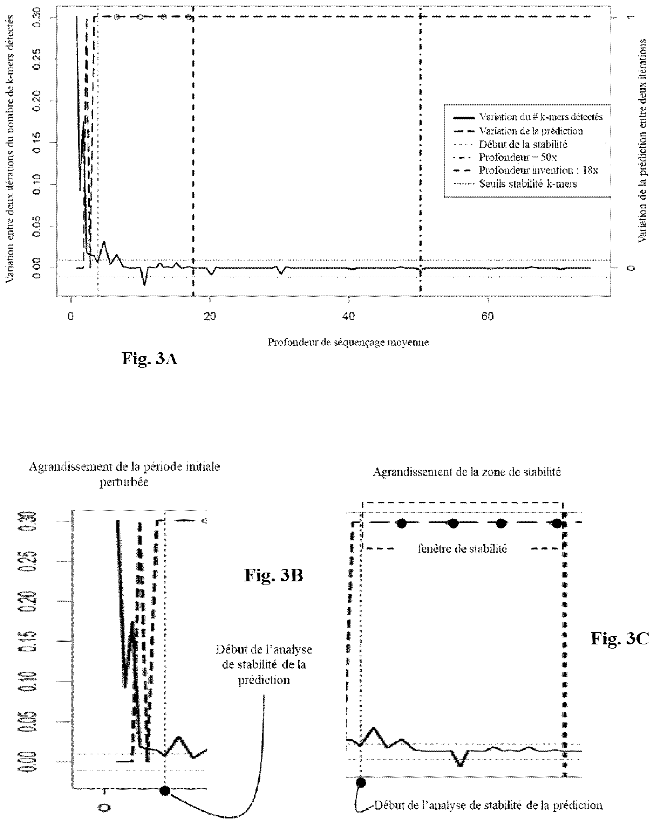

La **figure 3** illustre une prédiction bi-classes correspondant à la résistance ou la sensibilité d'une souche d'espèce bactérienne donnée à un antibiotique donné, avec un zoom sur la période initiale d'instabilité et un zoom sur la fenêtre d'analyse de stabilité de la prédiction.

### PARAMETRAGE DE L'INVENTION

Pour détecter au plus tôt la stabilité de la prédiction, l'invention dispose donc des paramètres suivants qui peuvent être paramétrés seuls ou en combinaison :
- la manière dont sont détectés les k-mers, notamment la fraction X^{kmer} ; et
- la manière dont est détectée la stabilité de prédiction, notamment le seuil S^{Pred}, la largeur de la fenêtre de stabilité N^{pred} et l'accroissement ΔX entre deux itérations successives de la profondeur de séquençage moyenne.

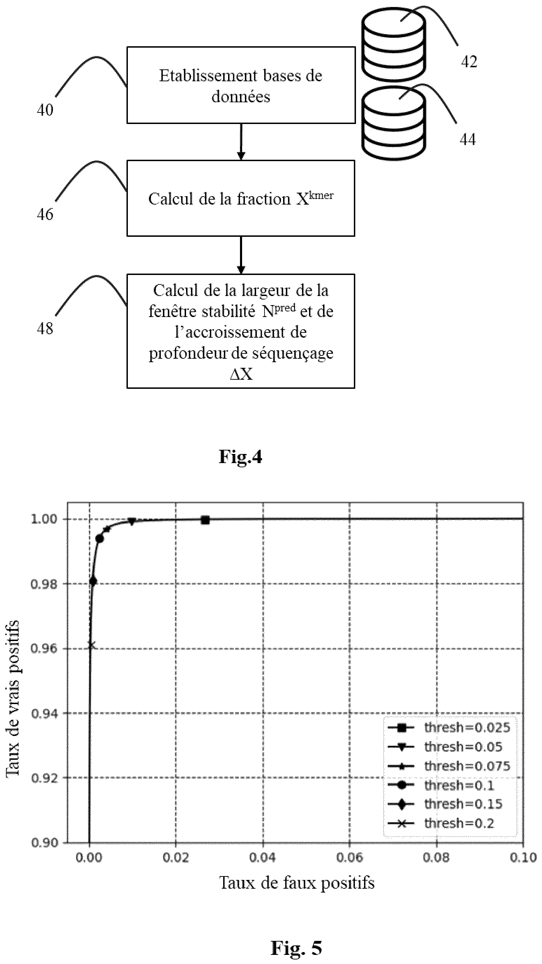

Il est à présent décrit, en relation avec la **figure 4****,** un procédé de détermination de la valeur desdits paramètres. Ce procédé débute, en **40,** par l'établissement d'une base de données **42,** dite de « vérité génomique », et une base de données de reads **44.** Ce procédé est mis en oeuvre par une unité informatique, par exemple le serveur **2002.** Plus particulièrement, pour chaque souche d'un ensemble de souches bactériennes préalablement collectées :
i. un génome de référence est déterminé. Préférentiellement, le génome de référence est le résultat d'un traitement visant à réduire au maximum, et de préférence annuler, les erreurs de séquençage. Par exemple, chaque souche est séquencée avec un séquenceur reads courts de la société Illumina avec une grande profondeur de séquençage (au moins 50x, de préférence supérieure à 100x) et un assemblage est mis en oeuvre. Cet assemblage de reads courts mène à un taux d'erreur très faible, voire nul à l'aide des outils d'assemblage actuels. Le génome assemblé est alors traduit en k-mers (ci-après « k-mers de référence ») en glissant une fenêtre de longueur k, par pas de 1, sur les contigs de la manière décrite précédemment et les k-mers de référence sont mémorisés dans la base **42.** Lorsque la prédiction phénotypique utilise un génome de référence, ce dernier peut être celui utilisé pour la détermination de la valeur des paramètres.
ii. le génome de la souche est séquencé avec un séquenceur du même type que celui utilisé pour l'analyse microbiologique (même type de plateforme, de flow cell et de préparation de librairie et de base calling que ceux utilisé par la caractérisation phénotypique) de manière à produire des paquets de reads, et ce pour une profondeur de séquençage importante (au moins 50x, de préférence supérieure à 100x). Ces paquets de reads sont optionnellement filtrés puis traduits en k-mers (ci-après « k-mers ONT ») et mémorisés dans un ensemble de k-mers de la manière décrite précédemment. La base **44** mémorise donc des itérations de l'ensemble de k-mers ONT, et donc un ensemble de k-mers ONT qui s'accroit en fonction de la profondeur de séquençage.

Le procédé se poursuit, en **46,** par le réglage de la fraction X^{kmer}, et donc du nombre de copie qu'un read doit avoir dans l'ensemble de k-mers pour conclure à sa détection.

A cette fin, pour chaque souche bactérienne préalablement collectée, et pour chaque itération de l'ensemble de k-mers ONT, une courbe ROC est calculée en fonction de plusieurs valeurs de la fraction X^{kmer}, cette fraction étant par exemple incrémentée, depuis 0, par pas régulier (par exemple 0.005). Pour chacune des valeurs de cette fraction, des k-mers ONT de l'ensemble de k-mers sont jugés détectés ou non. Les k-mers ONT détectés sont alors comparés aux k-mers de référence et si un k-mers ONT détecté est un k-mer de référence, alors il s'agit d'un vrai positif et sinon un faux positif.

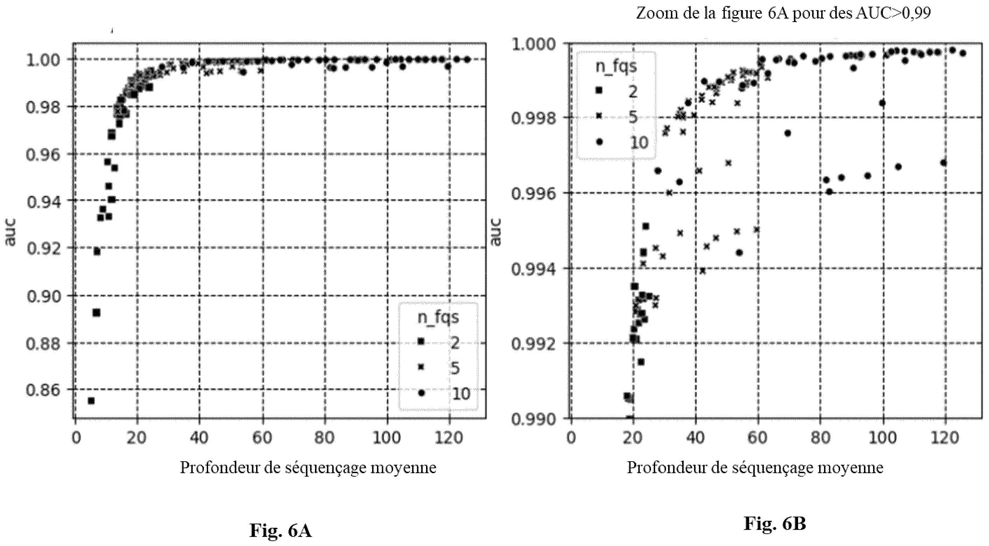

La **figure** 5 illustre la courbe ROC pour une souche donnée et pour différentes valeurs de X^{kmer} (nommé « Thresh » dans la figure). Pour chacune des courbes ROC, la valeur de la fraction X^{kmer} maximisant la surface sous la courbe ROC (surface notée « AUC ») est mémorisée (dans l'exemple de la figure 5, la valeur Thresh = 0,075). Il est ainsi obtenu pour chaque souche un ensemble de valeurs optimales de la fraction X^{kmer} en fonction des itérations. Les **figures 6A** et **6B** illustrent des valeurs d'AUC pour les valeurs optimales de la fraction X^{kmer} pour un ensemble de 55 souches bactériennes (dans l'exemple des souches de *Staphylococcus aureus*) en fonction de la profondeur de séquençage moyenne, 3 itérations (notées « *n_fqs* ») étant représentées.

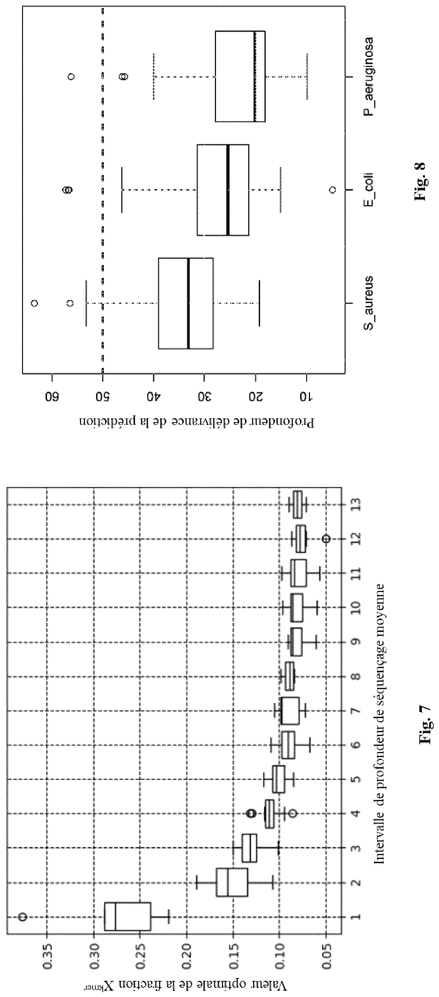

Les valeurs optimales la fraction X^{kmer} sont ensuite regroupées par intervalle de profondeurs de séquençage, par exemple des intervalles de 10x, de manière à obtenir une distribution de ladite fraction. Une telle distribution pour les 55 souches bactériennes est illustrée à la **figure 7** sous forme de diagramme en blocs (« box plot ») en considérant la médiane et le 5^{e} et 95^{e} centiles. Le chiffre 1 des abscisses correspond à l'intervalle de profondeurs de séquençage moyenne [0x ; 10x[, le chiffre 2 correspondant à l'intervalle [10x ; 20x[, etc.

Les valeurs médianes à partir de l'intervalle 2, et donc pour des profondeurs de séquençage moyenne supérieure à 10x, définissent un intervalle de fractions optimales selon l'invention, en particulier l'intervalle [0,08 ; 0,18], dont les valeurs mènent à des AUC supérieures à 0,99.

Le procédé se poursuit, en **48,** par la détermination de la largeur de la fenêtre de stabilité N^{pred} et l'accroissement ΔX entre deux itérations successives de la profondeur de séquençage moyenne. Plus particulièrement, cette étape consiste à calculer des valeurs pour ces paramètres tel que le résultat de la prédiction phénotypique à la fin de la fenêtre stabilisée soit sensiblement identique à la prédiction phénotypique obtenue pour une profondeur de séquençage moyenne pour laquelle la prédiction est connue pour être stabilisée. Par exemple, la prédiction phénotypique à une profondeur de séquençage moyenne supérieure ou égale à 50x, par exemple 50x, est choisie comme référence. Il est ensuite défini un espace de recherche constitué de valeurs comprises dans l'intervalle de fractions optimales X^{kmer}, par exemple une discrétisation dudit intervalle par pas de 0,05, de valeurs largeur N^{pred}, par exemple les entiers compris entre 1 et 10, et de valeur de l'accroissement de ΔX, par exemple de 1x à 10x par pas de 1x. Pour chaque triplet de valeurs X^{kmer}, N^{pred} et ΔX, la prédiction phénotypique est mise en oeuvre pour l'itération marquant la fin de la fenêtre de stabilité, et comparée à la prédiction phénotypique de référence. Cette comparaison est de préférence faite sur la base d'une métrique mesurant la pertinence de cette prédiction par rapport à la prédiction de référence, par exemple la balanced accuracy ou la moyenne des balanced accuracy si la prédiction est multi-classe.

Le tableau suivant illustre la balanced-accuracy pour une prédiction binaire (à savoir résistant ou non à un antiobitique) telle que décrite dans les documents les demandes WO2021180771 et WO2021180768 et appliquée aux k-mers des 55 souches de *S. aureus,* pour X^{kmer} = 0,1 et différentes valeurs de N^{pred} et ΔX.

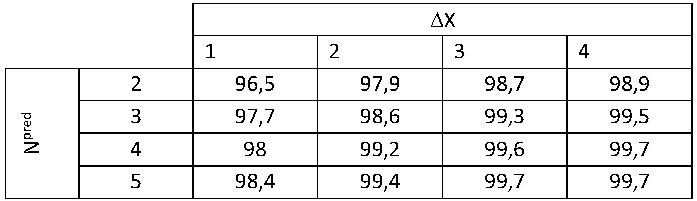

Les inventeurs ont noté que la valeur de la métrique atteint un plateau lorsque N^{pred} est supérieur ou égal à 4 et ΔX est supérieure ou égal à 3, et ce pour l'ensemble des valeurs de X^{kmer} dans l'intervalle [0,08 ;0.18].

La fenêtre de stabilité est ainsi avantageusement choisie tels que N^{pred}= 4 et ΔX ≥3X, de préférence ΔX ≤ 4X, ces couples de valeurs correspondant à la délivrance la plus rapide de la prédiction phénotypique. De manière encore plus préférentielle, ΔX =3X. En effet, le gain de performances pour 4X par rapport à 3X est marginal alors que ce gain implique une délivrance plus tardive.

La **figure 8** illustre sous forme de box plot (médiane, percentiles 5% et 95%) la profondeur de séquençage moyenne à laquelle est délivré la prédiction phénotypique selon l'invention avec X^{kmer} = 0, 1 ; N^{kmer}= 1 ; S^{kmer} = 0,1 ; N^{pred} = 4 ; S^{Pred}=0,1 et ΔX = 3X et ce pour 55 souches de S. *aureus,* 80 souches de *E. coli* and 80 souches P. *aeruginosa.* On observe un gain d'au moins 15x en termes de profondeurs de champs par rapport à une prédiction phénotypique délivrée à 50x et même un gain supérieur à 30x sur certaines espèces microbiologiques.

L'invention dispose également des paramètres concernant la manière dont est détectée la fin de la période initiale d'instabilité, notamment le seuil S^{kmer} et le nombre d'itérations N^{kmer}. Les inventeurs ont constaté que S^{kmer} = 0,1 et N^{kmer}= 1 permettent de détecter avec robustesse la fin de la période d'instabilité. D'autres valeurs ont été testées avec un gain marginal sur la vitesse de délivrance de la prédiction.

### EXTENSION DE L'ENSEIGNEMENT DU MODE DE REALISATION

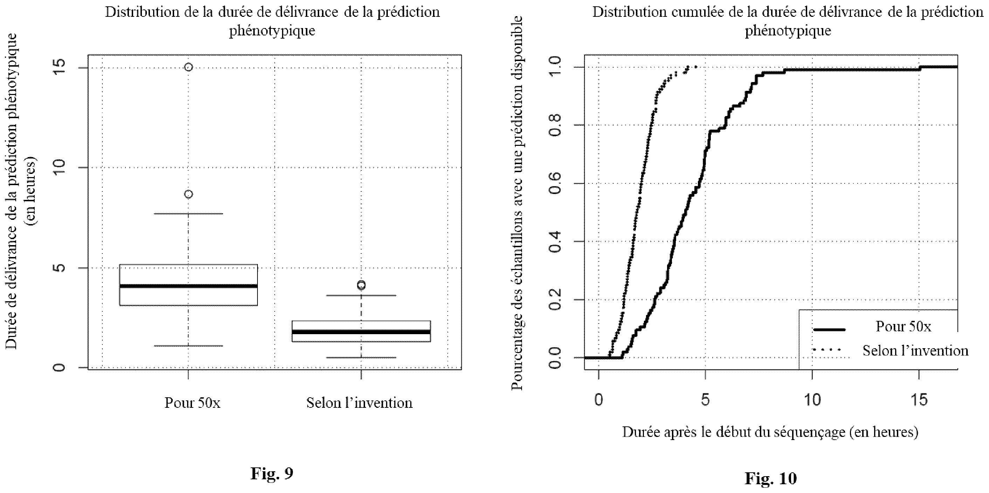

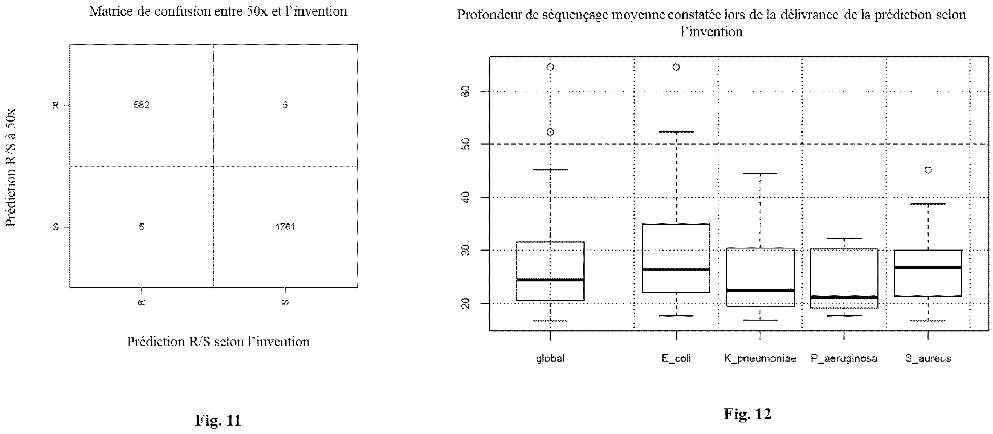

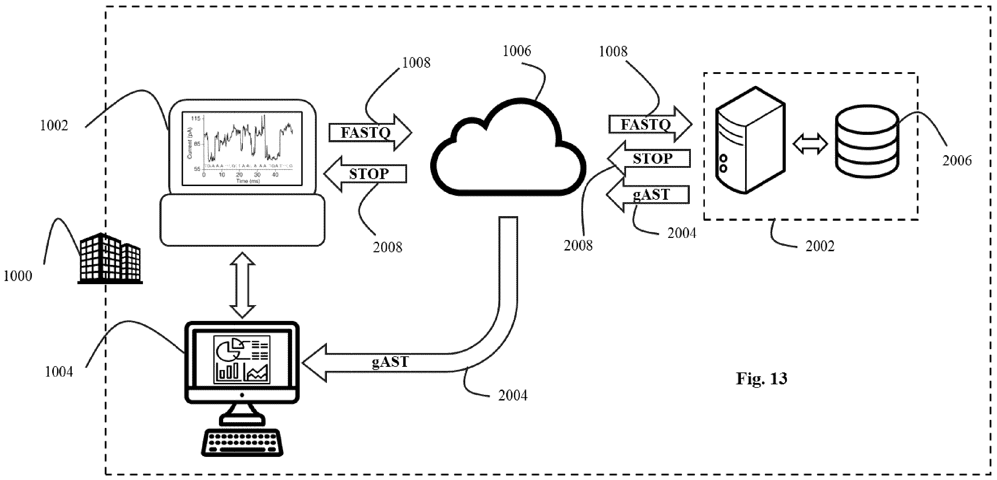

i. Il a été décrit une prédiction réalisée sur un isolat de souche bactérienne. L'invention s'applique également à la métagénomique, consistant à caractériser les microorganismes dans un même échantillons sans procéder à leur isolement comme décrit précédemment. La prédiction comprend par exemple une étape supplémentaire d'assignement des reads à une espèce particulière dont un ou plusieurs génomes sont mémorisés dans la base **2006,** étape connue sous le nom de « pooling ». Chaque pool de reads, assigné à une espèce, est alors traité de la manière précédente. Dans un tel cas, le séquençage est stoppé lorsque l'ensemble des prédictions sont stables. La **figure 9** illustre le gain de temps pour ces réglages entre une prédiction à 50x et une prédiction selon l'invention. On observe en moyenne un gain de temps de 3h. La **figure 10** illustre ce même phénomène pour les 215 souches testées décrites ci-avant pour la résistance/sensibilité à un antibiotique. On observe par exemple que 20% des prédictions sont délivrés quelques dizaines de minutes seulement après le début du séquençage, environ 70% des prédictions étant délivrées deux fois plus vite qu'une prédiction réalisée sur des reads à 50x. La **figure 11** est une matrice de confusion entre l'invention et une prédiction à 50x, et la **figure 12** illustre la profondeur de séquençage moyenne à laquelle la prédiction selon l'invention est détectée stabilisé et donc délivrée.
ii. Il a été décrit une prédiction phénotypique telle que décrite dans les documents WO2021180771 et WO2021180768. L'invention ne se limite pas à de telles prédictions. Par exemple, l'invention englobe la prédiction d'un résistome (i.e. la délivrance d'un ensemble de marqueurs génétiques suspectés d'être impliqués dans la résistance ou la sensibilité d'une souche à un antibiotique) ou d'un virulome par exemple.
iii. Il a été décrit une architecture matérielle et logicielle particulière pour la mise en oeuvre de l'invention. En particulier, les unités de traitement informatique et le ou les serveurs comprennent des mémoires informatiques (cache, RAM, ROM...) et un ou plusieurs microprocesseurs ou processeurs (CPU et/ou GPU), organisés ou non sous forme de noeuds de calcul, nécessaires à l'exécution d'instructions informatiques mémorisées dans les mémoires pour la mise en oeuvre du procédé selon l'invention. On comprendra que s'agissant de calcul tout type d'architecture informatique peut convenir et que la description ci-avant et ci-après ne doit pas être comprise comme limitant la portée de l'invention. Dans une variante (**figure 13**), l'architecture est hébergée par une seule organisation, par exemple un hôpital, le SaaS étant mis en oeuvre on-premise.

## Revendications

1. Procédé de prédiction d'un caractère phénotypique d'un organisme en fonction de son génome, comprenant :
• un séquençage dudit génome de manière à produire des séquences d'acides nucléiques numériques représentatives dudit génome, ci-après "reads", ledit séquençage étant configuré pour délivrer les reads a) en temps réel et/ou b) par lots à intervalles réguliers ou périodiques, lesdits reads étant mémorisés dans une mémoire informatique ;
• un traitement itératif mis en oeuvre par ordinateur, comprenant pour chaque itération:
- une mise à jour d'un ensemble de reads en fonction des reads délivrés en temps réel ou par lots ;
- un calcul d'une profondeur de séquençage dudit génome en fonction dudit ensemble de reads ;
- une détection de séquences d'acides nucléiques numériques de référence de longueur prédéterminée, ci-après "k-mers", dans ledit ensemble de reads, un k-mer étant détecté lorsque son nombre d'occurrences dans ledit ensemble est supérieure à une fraction prédéterminée de la profondeur de séquençage calculée;
- une prédiction du caractère phénotypique en fonction des k-mers détectés dans ledit ensemble de reads; et
- un calcul d'un critère d'arrêt et une mémorisation du caractère phénotypique prédit en tant que caractère phénotypique final lorsque ledit critère d'arrêt est atteint,
***caractérisé en ce que*** le calcul du critère d'arrêt comprend le calcul de la variation de la profondeur de séquençage calculée entre deux itérations, de la variation du nombre de k-mers détectés entre deux itérations et de la variation du caractère phénotypique prédit entre deux itérations, ***et en ce que*** le critère d'arrêt est atteint :
A. si la variation du nombre de k-mers détectés est stable pendant un premier nombre prédéterminé d'itérations ; et
B. à la suite de la stabilité de la variation du nombre de k-mers détectés pendant le premier nombre d'itérations :
B.1. si la variation du caractère phénotypique est stable pendant un second nombre d'itérations; et
B.2. pendant le second nombre d'itérations, si la variation de la profondeur de séquençage calculées entre deux itérations est supérieure à un seuil prédéterminé.

2. Procédé selon la revendication 1, ***caractérisé en ce que*** la fraction prédéterminée de la profondeur de séquençage est comprise entre 0,08 et 0,18.

3. Procédé selon la revendication 1 ou 2, ***caractérisé en ce que*** le premier nombre d'itérations est égal à 1.

4. Procédé selon l'une des revendications précédentes, ***caractérisé en ce que*** le second nombre d'itérations est supérieur ou égal à 4.

5. Procédé selon l'une des revendications précédentes, ***caractérisé en ce que*** le seuil est supérieur ou égal à 3x.

6. Procédé selon l'une des revendications précédentes, ***caractérisé en ce que*** la variation du nombre de k-mers détectés est stable lorsqu'elle est inférieure en valeur absolue à un premier seuil de stabilité, et la variation du caractère phénotypique est stable lorsqu'elle est inférieure à un second seuil de stabilité prédéterminé, ***et en ce que*** le premier et second seuil sont inférieurs ou égaux à 0. 1.

7. Procédé selon l'une des revendications précédentes, ***caractérisé en ce que*** séquençage est configuré pour délivrer des reads d'une longueur moyenne supérieure à 500kb.

8. Procédé selon l'une des revendications précédentes, ***caractérisé en ce que*** les reads ne sont pas assemblés.

9. Procédé selon l'une quelconque des revendications précédentes, ***caractérisé en ce que,*** préalablement au séquençage, le procédé comporte la production d'un échantillon à partir d'un isolat de microorganisme, le séquençage étant mis en oeuvre sur ledit échantillon.

10. Procédé selon l'une quelconque des revendications précédentes, ***caractérisé en ce que*** l'organisme est microorganismes, ***et en ce que*** le caractère phénotypique est un profil de résistance ou de sensibilité du microorganisme à au moins un anti-microbien.

11. Procédé selon la revendication 10, ***caractérisé en ce que*** le microorganisme est prélevé sur un patient ou un animal suspecté d'une infection microbienne, et ***en ce que*** le procédé comprend la détermination d'une thérapie antimicrobienne en fonction dudit profil et l'administration de ladite thérapie au patient ou à l'animal.

12. Procédé selon l'une quelconque des revendications précédentes, ***caractérisé en ce que*** lorsque le critère d'arrêt est atteint le séquençage est stoppé et/ou le caractère phénotypique final est affiché sur un écran.

13. Système de prédiction d'un caractère phénotypique d'un organisme en fonction de son génome, comprenant :
• un séquenceur d'acides nucléiques, ledit séquenceur étant configurée pour séquencer ledit génome de manière à produire des séquences d'acides nucléiques numériques représentatives dudit génome, ci-après "reads", ledit séquenceur étant configuré pour délivrer les reads a) en temps réel et/ou b) par lots à intervalles réguliers ou périodiques, lesdits reads étant mémorisés dans une mémoire informatique ;
• une unité de traitement d'information connectée au séquenceur pour recevoir lesdits reads et comprenant un ou plusieurs microprocesseurs et une mémoire informatique mémorisant des instructions informatiques qui, lorsqu'elles sont exécutées par le ou les microprocesseurs, mettent en oeuvre un traitement itératif, comprenant pour chaque itération:
- une mise à jour d'un ensemble de reads en fonction des reads délivrés en temps réel ou par lots ;
- un calcul d'une profondeur de séquençage dudit génome en fonction dudit ensemble de reads ;
- une détection de séquences d'acides nucléiques numériques de référence de longueur prédéterminée, ci-après "k-mers", dans ledit ensemble de reads, un k-mer étant détecté lorsque son nombre d'occurrences dans ledit ensemble est supérieure à une fraction prédéterminée de la profondeur de séquençage calculée;
- une prédiction du caractère phénotypique en fonction des k-mers détectés dans ledit ensemble de reads; et
- un calcul d'un critère d'arrêt et une mémorisation du caractère phénotypique prédit en tant que caractère phénotypique final lorsque ledit critère d'arrêt est atteint,
***caractérisé en ce que*** le calcul du critère d'arrêt comprend le calcul de la variation de la profondeur de séquençage calculée entre deux itérations, de la variation du nombre de k-mers détectés entre deux itérations et de la variation du caractère phénotypique prédit entre deux itérations, ***et en ce que*** le critère d'arrêt est atteint :
A. si la variation du nombre de k-mers détectés est stable pendant un premier nombre prédéterminé d'itérations ; et
B. à la suite de la stabilité de la variation du nombre de k-mers détectés pendant le premier nombre d'itérations :
B.1. si la variation du caractère phénotypique est stable pendant un second nombre d'itérations; et
B.2. si la variation de la profondeur de séquençage calculées pendant le second nombre prédéterminé d'itérations est supérieure à un seuil prédéterminé.

14. Système de prédiction selon la revendication 13, ***caractérisé en ce que*** la mémoire informatique mémorisant des instructions informatiques qui, lorsqu'elles sont exécutées par le ou les microprocesseurs, mettent en oeuvre un procédé selon l'une quelconque des revendications 2 à 12.

15. Produit programme d'ordinateur comprenant une mémoire informatique mémorisant des instructions informatiques qui, lorsqu'elles sont exécutées par un ou plusieurs microprocesseurs, mettent en oeuvre le traitement itératif selon l'une quelconque des revendications 1 à 12.

16. Procédé de production d'une mémoire informatique pour la prédiction d'un caractère phénotypique d'un organisme en fonction de son génome comprenant :
i. la mise à jour itérative d'un ensemble de séquences d'acides nucléiques numériques représentatives dudit génome, ci-après "reads", en fonction de nouveaux reads représentatifs dudit génome;
ii. l'exploration mise en oeuvre par informatique d'un espace défini par au moins un premier nombre d'itérations, un second nombre d'itérations et un seuil, ci-après « triplet », l'exploration comprenant pour chaque valeur d'un ensemble de triplets dudit espace (a) un calcul d'une profondeur de séquençage dudit génome en fonction dudit ensemble de reads, une détection de séquences d'acides nucléiques numériques de référence de longueur prédéterminée, ci-après "k-mers", dans ledit ensemble de reads, un k-mer étant détecté lorsque son nombre d'occurrences dans ledit ensemble est supérieure à une fraction prédéterminée de la profondeur de séquençage calculée, (b) une prédiction du caractère phénotypique en fonction des k-mers détectés dans ledit ensemble de reads, et (c) un calcul d'un critère d'arrêt, le calcul du critère d'arrêt comprenant le calcul de la variation de la profondeur de séquençage calculée entre deux itérations, de la variation du nombre de k-mers détectés entre deux itérations et de la variation du caractère phénotypique prédit entre deux itérations ;
iii. la mémorisation dans la mémoire informatique (a) d'un triplet en tant que premier nombre d'itérations, second nombre d'itérations, et seuil et (b) d'instructions informatiques qui, lorsqu'elles sont exécutées par un ou plusieurs microprocesseurs, mettent en oeuvre le traitement itératif selon l'une quelconque des revendications 1 à 12, lorsque ledit triplet rempli les conditions I et II suivantes:
I.
A. si la variation du nombre de k-mers détectés est stable pendant le premier nombre prédéterminé d'itérations ; et
B. à la suite de la stabilité de la variation du nombre de k-mers détectés pendant le premier nombre d'itérations :
B.1. si la variation du caractère phénotypique est stable pendant le second nombre d'itérations; et
B.2. si la variation de la profondeur de séquençage calculées pendant le second nombre prédéterminé d'itérations est supérieure au seuil,
II. lorsque la condition I est remplie :
C. 1. si les k-mers détectés sont également détectés dans un génome de référence de l'organisme ;
C.2. et si le caractère phénotypique détecté est identique à un caractère phénotypique de référence pour l'organisme.
